# EUROPEAN PATENT APPLICATION

(11) **EP 4 813 440 A2**
(43) Date of publication of application: **30.09.2026**
(21) Application number: 26199143.4
(22) Date of filing: 28.02.2020
(51) Int. Cl.: A61M 60/894

(54) **COLLAPSIBLE DEVICE FOR CIRCULATORY ASSISTANCE**

(30) Priority: 01.03.2019 NL 2022661
(62) Document number(s) of the earlier application(s) in accordance with Art. 76 EPC: 20708699.2 / 3 930 781
(71) Applicant: CardiacBooster BV, 6538 SZ Nijmegen (NL)
(72) Inventor: VAN DORT, Daniel Immanuel Michael, 6531BR Nijmegen (NL); LUDWIG, Florian Niklas, 1213 BS Hilversum (NL); HERMANS, Bob Petrus, 6511AV Nijmegen (NL); PEIJ, Koen Ronaldus Antonius Hermanus, 5049 CJ Tilburg (NL); THANNHAUSER, Jos, 6511 GE Nijmegen (NL)
(74) Representative: Tomkins & Co

(57) **Abstract**

A heart support device for circulatory assistance is provided, the device comprising: a chamber body comprising an outer wall and defining an internal volume (Vₓ) configured to receive a volume of fluid, wherein the chamber body extends from a proximal end to a distal end and is configured to be at least partially placed in a heart chamber; and a catheter extending from the proximal end of the chamber body, wherein the chamber body comprises an elongated portion having an outlet opening at the proximal end of the chamber body, the elongated portion configured to extend through a heart valve and into a vessel adjacent the heart chamber when the chamber body is placed in the heart chamber, the outlet opening being in fluid communication with an exterior volume in which the chamber body is disposed, wherein the chamber body further comprises an inlet opening in the outer wall of the chamber body to provide fluid communication between the internal volume of the chamber body and the exterior volume, and wherein the outer wall of the chamber body is configured to alternately collapse and expand between a first configuration in which the internal volume Vₓ=V₁ and a second configuration in which the internal volume Vₓ=V₂, wherein V₁ is larger than V₂, and thereby pump the fluid through the outlet opening.

## Description

### Field of the invention

The present invention relates to a device for circulatory assistance for supporting the function of the heart. More particularly, the present invention relates to an intra-lumen (e.g. intra-vascular or intra-cardiac) device for providing circulatory assistance by providing a chamber configured to alternately collapse and expand within a heart chamber or associated lumen.

### Background of the invention

Conditions that affect the ability of the heart to pump blood around the body are a major cause of mortality worldwide. Conditions that compromise or impair the heart's ability to pump blood effectively include cardiovascular diseases related to constriction of blood vessels and conditions that affect the heart wall, thus impacting its capacity to expel blood from the chambers of the heart. Conditions that affect the capacity of the heart wall to expel blood from the heart chambers include chronic heart failure, cardiac shock, or other conditions that cause weakening, distension or scarring of the heart wall. For patients suffering from such conditions, it may be necessary or desirable to provide mechanical circulatory assistance.

Devices for providing circulatory assistance are known in the art. For example, US Patent Publication No. US2013/184515 describes an intraventricular balloon device comprising a slender flexible catheter with an inflatable balloon provided near the distal end. The balloon can be periodically inflated and deflated and acts as a displacement body within the heart, displacing blood volume as it inflates (during during diastole), before deflating (during systole).

International Patent Publication WO2016/001218 describes a heart support device for circulatory assistance with an internal member configured to be disposed within a heart lumen. The device comprises a dynamic volume body that can be inflated and deflated periodically with the natural (or modified) rhythm of the heart to provide circulatory assistance. The internal member has a substantially stiff wall strengthening portion arranged to engage an inner wall surface of the heart and an inflatable dynamic member that is inflatable to assist pumping action of the heart.

Although the effect of both of these systems is to dispose a residual volume of blood from the heart lumen or chamber, such displacement devices do not provide the efficiency of natural cardiac motion. There is thus a need for an improved circulatory assistance device.

International Patent Publication WO2005/102414 discloses various embodiments of an intravascular pump, which may be a left ventricle assist device, comprising a wall defining a pumping chamber. The wall is supported by struts which are attached to or part of an actuation system to move the wall from an expanded position to a contracted position and back to operate the pump. The actuation system may be electrically activated shape memory alloy struts, electroactive polymeric struts, or may be a balloon, struts attached to a slidable member or other suitable system.

US Patent Publication US2016/143739 discloses a prosthetic ventricular heart system having two prosthesis parts with a prosthetic heart chamber connecting the two. The prosthetic parts each have a valve/tube configuration intended to replace the aortic and mitral valve of the heart. The prosthetic heart chamber is compressed by a cooperative mechanism.

US Patent Publication US9,623,163 discloses embodiments of a left-ventricular assist device (LVAD) which has a generally ellipsoidal capsule fitted into a patient's ventricle, formed of a cage or frame of shape-memory wire which can be twisted open and shut to expand and collapse a thin membrane, to inflate with the incoming blood during diastole and to contract and squeeze out the blood during systole.

International Patent Publication WO2018/075875 discloses a ventricular ejection device comprising an anchoring stent which is adapted to be fitted along a perimeter to a myocardium of the ventricle, and a recoiling part in physical coupling to the anchoring stent. The recoiling part extends from the perimeter of the anchoring stent to a center of the device, and is adapted to extend or recoil based on flow of blood into and out of the ventricle. The device embodiments as disclosed herein are all relating a single diaphragm style of device, which is anchored at its surrounding to the wall of the left ventricle.

US Patent Publication US5,169,378 describes an intraventricular assist pump. The pump comprises a body pump, or external chamber, having a double lumen wall, that is expansible and of variable rigidity, and a transvalvular segment, or flexible "neck" of the pump, that conforms itself to the situation or position of "open" or "closed" of the aortic or pulmonary valves and avoids the need of using a valve in the discharge of blood from the pump.

International Patent Publication WO98/18508 discloses a circulatory assist device having a housing and a pumping membrane, with a control chamber and a pumping chamber. The device is mounted on a catheter and collapsed to a sufficiently small diameter to allow insertion into the vascular system of a patient. An expansion mechanism in the device, such as a stent, can be expanded to hold the housing in an expanded, substantially rigid state, while control fluid is pumped into and evacuated from the control chamber to repeatedly deflect the pumping membrane. At least one opening is formed in the device, to allow vascular fluid to enter and exit the pumping chamber as the pumping membrane deflects. Introduction and evacuation of control fluid can be synchronized with the heart cycle of the patient. After use, the housing is contracted to a smaller diameter to allow withdrawal from the vascular system.

### Summary of the invention

The present invention seeks to provide an intra-lumen cardiac assist device that more closely approximates the natural function of the heart by providing a chamber that expands and collapses to vary its internal volume. The resulting device can function as an artificial mini-heart (comprising a single chamber) that mimics the movement of a heart chamber. The mini-heart can be disposed within a cardio vascular lumen (e.g. in a heart chamber or a large vessel such as the aorta) to pump blood from the lumen in which is it placed, through the circulatory system.

Accordingly, in a first aspect of the invention, there is provided an intra-lumen heart support device for circulatory assistance, the device comprising: a chamber body comprising an outer wall and defining an internal volume configured to receive a volume of fluid, wherein the chamber body extends from a proximal end to a distal end. The chamber body comprises an outlet opening at its proximal end, the outlet opening being in fluid communication with an exterior volume in which the chamber body is disposed; wherein the outer wall of the chamber body is configured to alternately collapse and expand between a first configuration in which the internal volume V*ₓ*=V₁ and a second configuration in which the internal volume Vₓ=V₂, wherein V₁ is larger than V₂, and thereby pump the fluid through the outlet opening. The outlet opening comprises a cross-sectional internal diameter, wherein the cross-sectional internal diameter of the outlet opening is less than a maximum cross-sectional internal diameter of the chamber body. The outlet opening can be narrower than the widest part of the chamber body. In other words, the outlet opening can be narrow compared the widest part of the chamber body (measured in a transverse direction, and at least when the chamber body is in the fully expanded configuration). The narrow outlet opening can provide directional flow by directing the ejected fluid towards the target valve or direction (e.g. towards the aortic valve). Optionally, the chamber body tapers (preferably continuously) from its widest point toward the outlet opening.

In at least some embodiments, the chamber body further comprises a deformable support structure configured to support the chamber body in an open configuration.

The chamber body can be biased into the second, collapsed configuration, and the device can further comprise an actuation system configured to expand the chamber body against its bias. Alternatively, the chamber body can biased into the first, expanded configuration, and wherein the device further comprises an actuation system configured to collapse the chamber body against its bias. Embodiments in which the chamber body is biased into an intermediate position are also encompassed, wherein an actuation system is provided to deform the chamber against its bias into the expanded and collapse configurations.

The actuation system can comprise a first actuation component, such as a catheter or wire, coupled to a proximal end of the chamber body at a proximal fixation point. A second actuation component, also possibly a catheter or a wire can be coupled to a distal end of the chamber body at a distal fixation point. In such embodiments, the first the actuation component can be configured for relative movement with respect to the second actuation component to move the proximal fixation point back and forth relative to the distal fixation point to alternately collapse and expand the chamber body.

The actuation system can also comprise first and second actuation components coupled to first and second fixation points respectively, wherein the actuation components are configured for rotational movement with respect to each other, to twist the chamber body along its longitudinal axis, thereby collapsing the chamber. The twisting actuation mechanism can be employed in embodiment in which the distance between the first and second fixation points varies (as described above) and in embodiments in which the distance between the first and second fixation points remains fixed.

The first actuation component and the second actuation component can be configured for translational movement towards and away from each other to vary the length of the chamber body.

The chamber body can comprise a concertina-folded outer wall that can be expanded and collapsed to vary the internal volume of the chamber body. The concertina body can be formed with a pre-folded semi-compliant membrane. It can optionally comprise support ribs to support the chamber body in a deployed configuration.

The actuation system can comprise one or more inflatable ribs, said ribs, upon inflation, being configured to act against the bias of the chamber body to expand or collapse the chamber body.

The device can comprise a catheter coupled to the chamber body, the catheter comprising an internal lumen in fluid communication with the inflatable ribs.

The device can also comprise an actuation system configured to collapse and expand the body, and wherein the actuation system comprises a noose extending around a circumference of the chamber body, and wherein the noose comprises a variable loop. The loop can be coupled to the support structure such that tightening the noose cinches the support structure at a cinch point to restrict the volume of the chamber body.

The chamber body can be biased into the expanded configuration, and the actuation system can comprise a noose configured to collapse the body. Alternatively, the chamber body can be biased into the collapse configuration, and wire can be fed into the loop of the noose to expand the chamber body against its bias.

Advantageously, at least one inlet opening (in addition to the outlet opening) can be provided in the outer wall of the chamber body to provide fluid communication between the interior volume of the chamber body and a volume exterior to the device. Optionally, the inlet opening is closed by a one way valve.

The outlet opening can also comprise a one-way outlet valve. The one-way outlet valve can comprise a collapsible neck or a duck-bill valve.

In at least some embodiments, the chamber body can comprise: a plurality of deformable ribs forming a support scaffold; and one or more flexible panels or membranes supported by the deformable ribs to form the chamber body. Optionally, the chamber body can comprises a plurality of overlapping panels, and at least one region of overlap in which adjacent panels are not secured to each other, thereby forming at least one inlet opening between the between overlapping panels.

The collapsible support structure can comprise a frame comprising one or more ribs, a lattice or wire frame work, a deformable mesh or any combination of the above. The support structure preferably comprises a shape memory material, e.g. a shape memory alloy such as nickel-titanium or nitinol.

The plurality of ribs can extend substantially parallel to the longitudinal axis or they may comprises a plurality of helically collapsible ribs.

The outlet opening can have a substantially constant cross-sectional diameter whilst the cross-sectional diameter of the widest part of the chamber body can vary as the chamber body expands and collapses. Alternatively, the cross-sectional diameter of the narrow opening can vary as the chamber body expands and collapses. In such embodiments, the narrow opening is advantageously narrower than the widest part of the chamber body at any given point in an expand-collapse pumping cycle.

In addition to the device described above, the present invention provides a method of providing circulatory assistance to a patient. Accordingly, in a second aspect of the invention, there is provided a method for providing circulatory assistance, the method comprising the steps of: providing an intra-lumen circulatory assist device within a cardiovascular lumen, the device comprises a chamber body having an outer wall defining and internal volume, and an outlet opening providing fluid communication between the interior volume of the chamber body and a volume external to the chamber body. The method further comprises the step of, with an actuator, alternately collapsing and expanding the outer wall of the device to varying the internal volume of the chamber body between a first internal volume, and a second internal volume being greater than the first internal volume to thereby provide circulatory assistance.

Further embodiments of the present invention will be apparent from the dependent claims and the following description and drawings.

### Brief description of the drawings

Fig. 1 shows a heart support device according to the present invention, disposed within a heart chamber;
Figs. 2A and 2B show the expanded and collapsed configurations of a heart support device according to a first embodiment of the invention;
Fig. 2C shows an alternative collapsed configuration of the heart support device according shown in Fig. 2A;
Figs. 3A and 3B show the expanded and collapsed configurations of a heart support device according to a second embodiment of the present invention in two states;
Figs. 4A and 4B show the expanded and collapsed configurations of a heart support device according to a third embodiment of the present invention in two states;
Figs. 5A and 5B show the expanded and collapsed configurations of a heart support device according to a fourth embodiment of the present invention in two states;
Figs. 5C and 5D show the expanded and collapsed configuration of a heart support device according to a fifth embodiment of the present invention;
Figs. 6A and 6B show the expanded and collapsed configurations of a heart support device according to a sixth embodiment of the present invention in two states;
Fig. 7A shows a cross-sectional view of an exemplary embodiment of a heart support device comprising a chamber body according to a first construction;
Fig. 7B shows side view of the exemplary embodiment shown in Fig. 7A;
Fig. 8A shows a side view of an exemplary embodiment of a heart support device comprising a chamber body according to a second construction;
Fig. 8B shows a cross-sectional view of the exemplary embodiment shown in Fig. 8A with the chamber body in an expanding phase;
Fig. 8C shows a cross-sectional view of the exemplary embodiment shown in Fig. 8A with the chamber body in a collapsing phase;
Fig. 9A shows a side view of an exemplary embodiment of a heart support device comprising a chamber body according to a third construction.
Fig, 9B shows a cross-sectional view of the exemplary embodiment shown in Fig. 9A with the chamber body in an expanding phase;
Fig. 9C shows a cross-sectional view of the exemplary embodiment shown in Fig. 8A with the chamber body in a collapsing phase;
Figs. 10A and 10B show the expanded and collapsed configurations of a heart support device according to an embodiment of the invention comprising an alternative support and actuation arrangement.

### Detailed description of the drawings

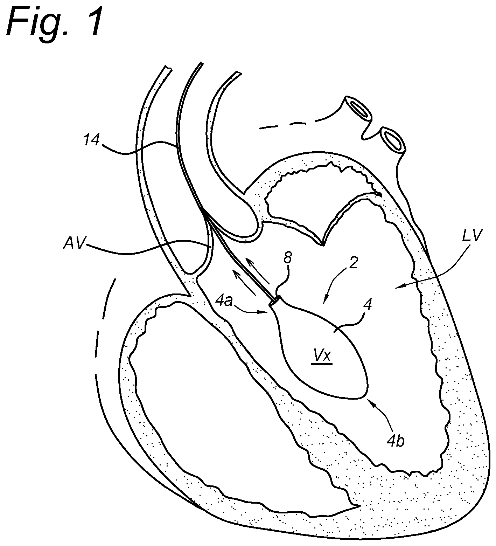

Fig. 1 shows an exemplary embodiment of a heart support device 2 for providing circulatory assistance. The heart support device 2 shown in Fig. 1 is configured to sit within a cardiovascular lumen (e.g. a chamber of the heart or a large vessel such as the aorta). In the illustrated embodiment, the device 2 is configured for deployment in the left ventricle LV and is configured to pump blood toward the aortic valve AV. The device 2 is advantageously mounted or connected to at least one catheter 14 with which the device 2 is delivered and deployed. The device 2 generally comprises a chamber body 4 comprising an outer wall that encloses an internal volume Vx, which is in fluid communication with its surroundings by way of an outlet opening 8. The outlet opening 8 is located at the proximal end 4a of the device. The distal end is denoted by reference numeral 4b.

The chamber body 4 is configured to alternately collapse and expand between a first configuration having a first internal volume wherein Vx=V1 and a second configuration having a second internal volume, wherein Vx= V2. The internal volume V2 of the chamber body 4 in the collapsed configuration is smaller than the internal volume V1 of the expanded configuration.

As the internal volume of the chamber body varies due to deformation of the outer wall, blood is expelled through the outlet opening 8 to provide circulatory assistance. As the internal volume of the chamber increases with expansion of the chamber body 4, blood is drawn into the internal volume of the chamber body 4; as the internal volume of the chamber decreases as the chamber is collapsed, blood contained within the chamber is ejected through the outlet opening 8, in this case towards the aortic valve. Expansion of the chamber body 4 during diastole and contraction of the chamber body 4 during systole can thus provide circulatory assistance by pumping blood from the heart chamber (in the illustrated embodiment the left ventricle LV) towards the target valve (in the illustrated case the aortic valve AV).

In the embodiment shown in Fig. 1, the device 2 can be advanced into the heart chamber in a minimally invasive manner through the aortic valve. The catheter 14 thus extends from the proximal end of the device. However, it will be appreciated that the present invention can be adapted for placemen in other heart chambers or associated lumens, including large vessels, such as the aorta, in which a distally extending catheter may be more convenient. The present invention thus also encompasses embodiments in which the catheter 14 extends from the distal end of the device.

As used herein, the term "proximal" refers to the end of the chamber comprising the outlet opening 8 through which blood is ejected from the chamber, and does not imply any particular orientation of the device. Moreover, although embodiments of the present invention are described herein with reference to device in situ as shown in Fig. 1 (wherein the device is located in the left ventricle LV), the skilled person will appreciate that the present invention may also be implemented in devices adapted for placement in other heart chambers (e.g. the right ventricle), or for placement outside the heart (e.g. in the aorta).

To provide directional flow towards a target valve or in a predetermined direction, the device 2 can comprise a neck or narrow opening at its proximal end that forms the outlet opening 8. The outlet opening 8 can thus have a cross-sectional area (measured in a transverse direction, i.e. in the plane orthogonal to the axis of the catheter 14), that is less than the transverse cross-sectional area of the chamber body.

The outlet opening 8 can provide the only fluid communication between the internal volume of the chamber and a volume external thereto, such that fluid in drawn into the internal volume of the chamber through the outlet opening 8 as the chamber body 4 expands, and expelled through the same aperture as the chamber body 4 is collapsed.

However, in some embodiments of the invention, the chamber body 4 is further provided with one or more inlet openings (not shown in Fig. 1) configured to allow the ingress of blood through the chamber walls into the interior volume of the chamber. In such embodiments, the inlet openings can be configured as a one-way valve that allows the flow of blood into the chamber, whilst preventing the flow of blood in the opposite direction.

The outlet opening 8 may also comprise a one-way valve structure, such that the internal volume of the chamber is in fluid communication with a volume exterior thereto via the one-way valve. Such a configuration can allow fluid to be ejected from the internal volume of the chamber through the outlet opening 8 toward the target valve (e.g. the aortic valve), whilst preventing or limiting the flow of fluid in the opposite direction. Optionally, the one-way inlets can be configured as apertures between overlapping panels forming the chamber body. Additional details of such an embodiment will be provided below.

In embodiments comprising a one-way valve structure at the opening 8, when the chamber body expands (increasing the internal volume), blood is drawn from the heart chamber in which the device is positioned, through the inlet openings in the chamber wall, and into the internal volume of the chamber. The one-way valve structure limits or prevents the flow of blood into the chamber through the opening 8. Conversely, as the chamber body 4 is collapsed, blood is ejected through the one-way valve at the outlet opening 8 towards the aortic valve AV, thereby providing circulatory assistance. Optionally, the outlet opening 8 can comprise a collapsible tube. For example, the outlet opening can comprise a duckbill valve comprising a collapsible tube or neck that is supported at one end in an open configuration, but allowed to adopt an unsupported flattened configuration at the opposite end.

The collapsible tube or neck can be dimensioned such that the open end sits within the chamber of the heart in which the device is located. Alternatively, the neck can be elongated such that it extends through a valve, e.g. the aortic valve, such that the opening of the neck is located in a different chamber or vessel, e.g. the aorta.

By providing a deformable chamber body 4 that can be alternately collapsed and expanded as described above, the present invention can provide circulatory assistance in a manner that more closely mirrors the natural function of the heart. As will be understood from the following disclosure, the deformation of the outer wall of the chamber can be realised in different ways. However, due to the change in shape of the wall of the chamber, the volume change can be harnessed to provide circulatory assistance for patients with sub-optimal heart function.

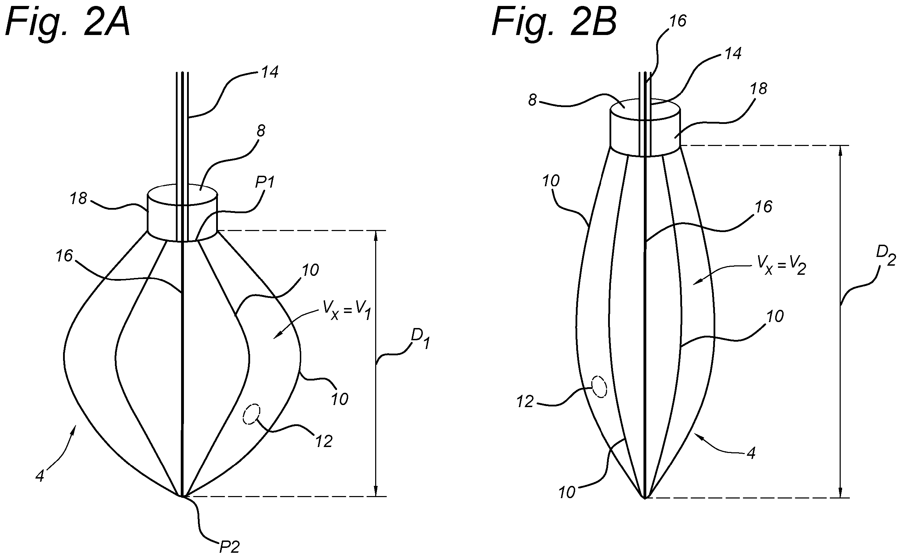

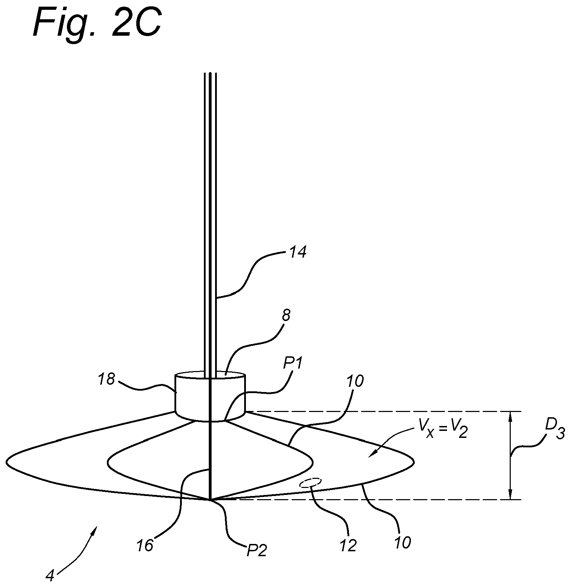

Several exemplary embodiments of the invention will now be described with reference to Figs. 2-10B, each of which can provide an effective way of providing an intra-cardiac or intra-lumen circulatory assistance, and can be deployed and controlled in a simple mechanical manner. These advantages, and further benefits provided by the present invention will be apparent from the description of the exemplary embodiments below.

Figs. 2A and 2B show a first embodiment of the invention in which the internal volume of the chamber is alternately varied by varying the distance between the proximal and distal ends. As shown in Figs. 2A and 2B, by varying the distance between the proximal and distal ends of the chamber, the shape of the chamber varies between an expanded configuration having a first (larger) internal volume V1 (see. Fig. 2A), and an elongate configuration having a second (smaller) internal volume V2.

The device 2 comprises a chamber body 4 having an outer wall which defines the internal volume Vx. An outlet opening 8 is provided at a proximal end of the chamber body 4. The chamber body 4 comprises a deformable support structure or scaffolding, which supports a membrane 12. In the drawings accompanying this application, the membrane 12 is generally depicted as a transparent or semi-transparent membrane to more clearly illustrate the internal components of the device. However, non-transparent membrane materials may be used.

In the embodiment shown in Figs. 2A-2B, the support structure 10 comprises a plurality of ribs extending between the proximal and distal ends of the device 2. The ribs 10 are formed of a resiliently or elastically deformable material, such as a shape memory alloy, (e.g. nitinol), or from any other resiliently deformable metal, alloy, polymer or other suitable material. Other suitable materials for the support structure 10 will be apparent to the person skilled in the art in light of the present disclosure.

The membrane 12 extends between or over the support structure 10 to form the walls of the chamber body 4. The membrane 12 is formed of a material that is substantially impermeable to liquid and can comprise a semi-compliant material such as nylon, polyurethane, polyether block amides (Pebax), polyethylene terephthalate (PET) or similar materials.

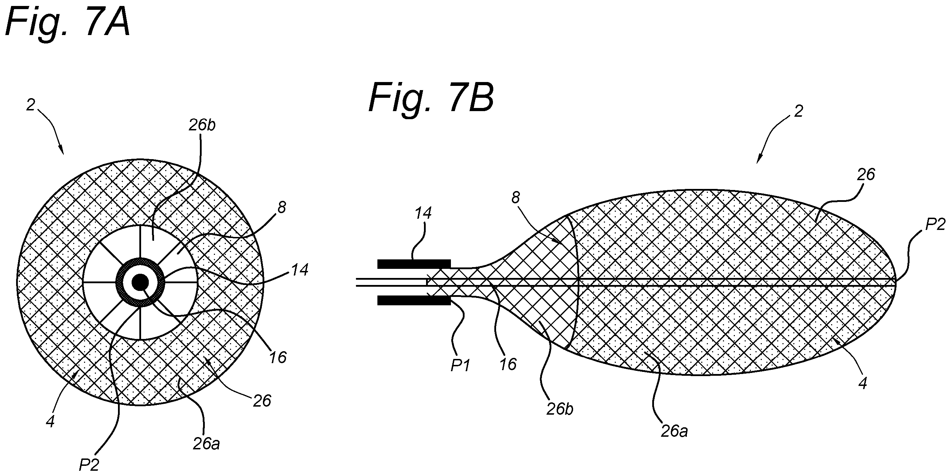

The support structure 10 comprises ribs that extend in a longitudinal direction from the proximal end of the chamber body 4 to the distal end of the body. The ribs 10 preferably converge or are connected to each other at their proximal ends and at their distal ends respectively. In at least one example, the ribs may be joined to a support ring (see end view in Fig. 7A).

A catheter 14 is fixed to the chamber body 4 (and preferably the support structure 10) at a proximal fixation point P1. The catheter 14 is configured to support the device 2 within the heart chamber (see Fig. 1). In the embodiment shown in Figs. 2A and 2B, the catheter 14 comprises an internal lumen through which a wire 16 (e.g. a pull-wire or a push-wire) extends. The wire 16 is coupled to the distal fixation point P2. The wire 16 is configured for relative axial movement with respect to the catheter 14, such that the distal fixation point P2 can be pulled toward (and/or pushed away from) the proximal fixation point P1. Since the ribs 10 are connected to the first and second fixation points, decreasing the distance Dx between P1 and P2 causes the ribs 10 to bend outwardly (away from the axis defined between P1 and P2), thereby expanding the chamber body 4 to the configuration shown in Fig. 2A. Conversely, increasing the distance between P1 and P2 causes the ribs 10 to straighten, forcing the chamber body to adopt an elongate shape (as shown in Fig. 2B). Since the internal volume of the elongate shape shown in Fig. 2B is less than the internal volume of the radially expanded shape shown in Fig. 2A, increasing the distance Dx between P1 and P2 leads to blood being expelled through the outlet opening 8. Circulatory assistance can thus be provided by alternately increasing and decreasing the distance between P1 and P2 to pump blood through the outlet opening 8.

In at least some exemplary embodiments of the invention, the length between the first and second fixation points P1 and P2 can vary between 3 and 7 cm (in the expanded and collapsed configuration). This can lead to a resulting variation in the maximum diameter of the chamber body (measured perpendicular to the longitudinal axis) of between 0.3 cm and 5 cm. It will be appreciated that the maximum and minimum lengths (and the maximum and minimum diameters) can be varied depending on the anatomy of the patient. For example, much smaller devices can be made for children, and larger devices can be made for large adults. Generally, it is advantageous for the collapsed diameter to be as small as possible, such that the volume with the chamber body is as small as possible when the length of the body is maximum. For example, the collapsed diameter may be only slightly larger than the catheter 16. For example, the collapsed diameter may be between 3-5 mm. The skilled person will appreciate that these dimensions are provided as an example of a device suitable for use in many patients, but the invention is not limited to these dimensions and the embodiments and modes of operation described herein is applicable to devices having larger and smaller dimensions.

The wire 16 can be configured as a pull-wire, a push-wire or both, depending on the at-rest, un-deformed shape of the chamber body 4. For example, the chamber body 4 can be configured such that the ribs 10 can be formed of a resiliently deformable material that adopts the shape shown in Fig. 2A with the influence of an external force. In such an embodiment, the wire 16 can be configured as a push-wire to push the distal fixation point P2 away from the proximal fixation point P1, against the bias of the ribs 10. The chamber body 4 can then return to its un-deformed (expanded) state due to the resilience of the ribs, before being extended again against its bias by pushing the push-wire 16 in a distal direction with respect to the catheter 14.

In alternative embodiments, the device 2 can be configured such that the ribs 10 bias the chamber body 4 into the elongated configuration shown in Fig. 2B. In such embodiments, the wire 16 can be configured as a pull-wire, configured to pull the distal fixation point P2 towards the proximal fixation point P1, against the bias of the ribs. Upon release of the pull-wire 16, the ribs relax to their un-deformed position (Fig. 2A) and the chamber body 4 returns to the expanded configuration.

Of course, it will be appreciated that the ribs 10 can be biased into an intermediate position (between Fig. 2A and Fig. 2B) and the wire 16 can act as a pull-wire and a push-wire, reciprocating back and forth to expand and collapse the chamber body 4. Even if the support ribs are not biased into the expanded or elongated position shown in Figs. 2A and 2B, in at least some embodiments, the ribs 10 can be resiliently deformable so that the device 2 can be collapsed completely to allow minimally invasive placement, before deployment to the active configuration(s) shown in Figs. 2A and 2B.

In the embodiment described above, the relative movement between the proximal fixation point P1 and the distal fixation point P2 is described with P1 maintaining a fixed position within the heart chamber, and P2 moving relative to P1. However, it will be appreciated that relative movement between the first and second fixation points can also be achieved by maintaining P2 in a fixed position within the heart chamber (or an associated vessel), whilst P1 is moved back and forth relative to P2. Equally, it is possible that neither P1 nor P2 maintain a fixed position within the heart chamber whilst relative movement between P1 and P2 occurs. It will therefore be understood that relative movement as defined in the context of the present invention can be achieved between P1 and P2 in each of these scenarios.

Depending on the precise configuration of the device and its placement within the heart chamber (or associated vessel), it may be preferred to maintain the proximal fixation point P1 in a (semi) stable position within the heart to ensure a stable position of the outlet opening 8, relative to the target valve (e.g. the aortic valve AV, as shown in Fig. 1). This configuration may be particularly advantageous in embodiment that comprise one or more one-way inlet valves, through which blood can be drawn into the internal volume of the chamber as the chamber body 4 expands.

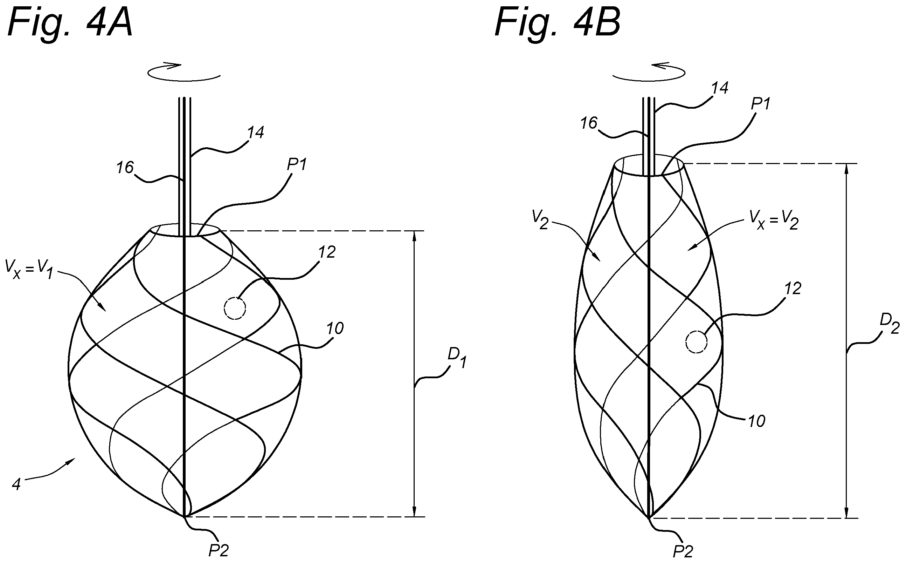

As shown in Figs. 2A and 2B, the chamber body 4 preferably has its maximum diameter (when taking a transverse cross-sectional view along line C-C) in a middle portion of the chamber body 4, between the proximal and distal ends, preferably in the middle half or third of the chamber body 4. As described above with reference to Fig. 1, the proximal outlet opening 8 has a cross-sectional diameter that is smaller than the maximum diameter of the chamber body 4. The relatively small cross-sectional diameter of the outlet opening can provide improved directional flow of fluid ejected from the outlet opening, e.g. towards the aortic valve. In the embodiment shown in Figs. 2A and 2B, the device 2 comprises a neck 18. However, the device 2 shown in Figs. 2A and 2B does not require neck 18 to provide relatively narrow outlet opening 8. Instead, the opening 8 can be formed as an opening in the membrane at the proximal end of the chamber body 4 (see, for example, the opening 8 in Figs. 4A and 4B).

In at least some embodiments (not shown in the illustrated embodiments), the opening 8 is not relatively narrow compared to the internal diameter of the body. Instead, the outlet opening 8 can be the widest portion of the chamber body 4, or equal in diameter to the widest portion. Moreover, the outlet opening 8 may be configured to expand and collapse with the alternately expanding and collapsing body.

As illustrated in Fig. 2C, instead of being collapsed from an expanded configuration (shown in Fig. 2A) to an elongate configuration (shown in Fig. 2B) to reduce the internal volume of the chamber body, the device 2 can be configured to collapse to a flattened configuration, as shown in Fig. 2C. In such a configuration, the internal volume of the chamber body is decreased by bringing P1 and P2 into close proximity to flatten the chamber body.

It will be appreciated that as a consequence of a sphere having the largest volume for a given surface area, the device 2 defines its maximum internal volume when the chamber body 4 approximates (as closely as possible) a spherical shape. As the chamber body 4 deforms away from the maximum (either towards an elongate shape - shown in Fig. 2B - or a flattened shape - shown in Fig. 2C), the internal volume of the chamber body is reduced. Thus, according to embodiment of the present invention, the chamber body 4 can be collapsed by bringing the first and second fixation points P1 and P2 into close proximity such that Dx approaches a minimum possible distance, or by moving the first and second fixation points P1 and P2 away from each other, such that Dx approaches a maximum possible distance.

Although this flattened configuration is described only with reference to Figs. 2A-2C, the skilled person will appreciate that collapsing the chamber by flattening the chamber body 4 is applicable to all embodiments of the present invention. Moreover, in such embodiments, the length of the chamber (e.g. the distance between P1 and P2 can vary between approximately 0cm and 7cm, whilst the diameter of the chamber body varies between approximately 0.3 cm and 7cm. The skilled person will appreciate that these dimensions are provided as an example of a device suitable for use in many patients, but the invention is not limited to these dimensions and the embodiments and modes of operation described herein is applicable to devices having larger and smaller dimensions.

As discussed with respect to Fig. 1, the opening 8 can be configured to allow egress of fluid (blood) from the internal volume of the chamber body 4 through the outlet opening 8, whilst preventing or limiting the flow of fluid in the opposite direction (i.e. into the chamber). The neck 18 can thus be supported in an open configuration at its connection to the chamber body 4 (the distal end of the neck 18), with the proximal end of the neck being configured to collapse in on itself. By providing a collapsible neck 18, which is made of a flexible tube, a duckbill-type valve can be provided at the opening 8, which allows fluid to be ejected through the opening 8, whilst preventing or limiting the flow of blood in the opposite direction.

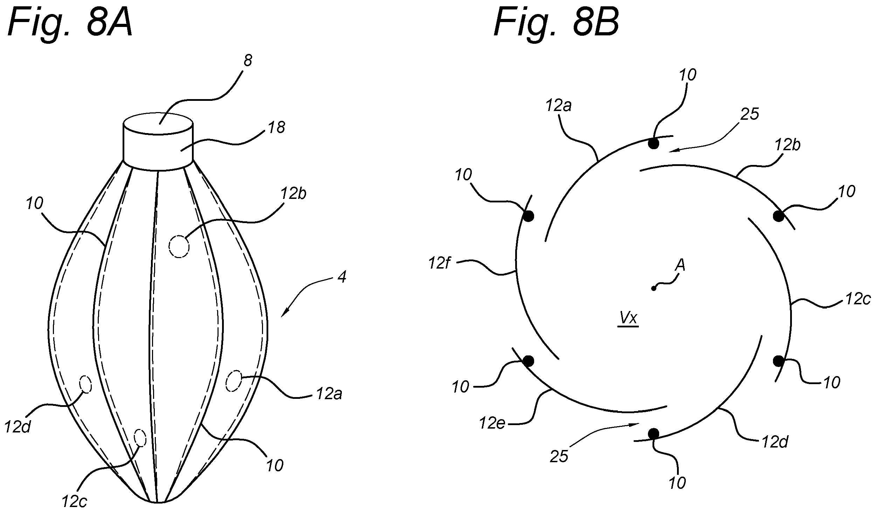

Although not shown in Figs. 2A and 2B, the chamber body 4 may further comprise one or more inlet openings through which blood can enter the internal volume of the chamber. The inlet openings can be configured to allow blood to enter the internal volume of the chamber as the chamber expands, whilst preventing egress of the blood from the chamber through the inlet openings when the chamber contracts. One exemplary inlet opening configuration will be discussed with reference to Figs. 8A and 8B, although the skilled person will appreciate that other one-way valve arrangements can be provided.

Moreover, although the illustrated embodiment comprises a catheter 14 affixed to the proximal fixation point P1 and the pull/push-wire 16 affixed to the distal fixation point P2, the skilled person will appreciate that the actuation mechanism can alternatively include two catheters arranged coaxially or in parallel, or two wires arranged in parallel. Where two coaxial catheters are used, the device 2 can advantageously be advanced along a guidewire extending along the inner lumen of the inner catheter.

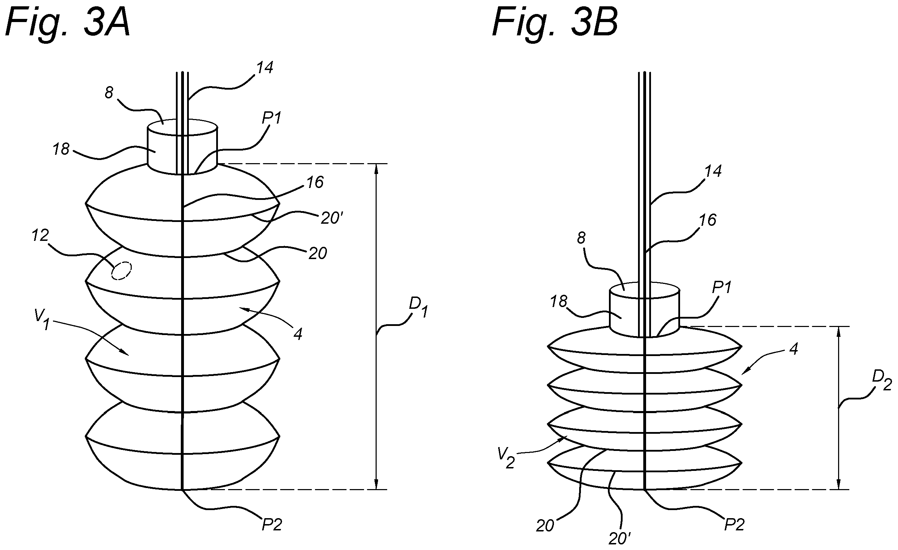

A second embodiment of the invention, which also varies the internal volume of the chamber by varying the distance between the distal and proximal fixation point P1, P2 of a chamber body, is illustrated in Figs. 3A and 3B. As shown in Figs. 3A and 3B, the chamber body 4 comprises an outer wall comprising a concertina or folded configuration that is configured to be extended and compressed as shown to pump blood through the outlet opening 8, acting in a similar manner to a bellows. The chamber body can comprise a deformable scaffold or mesh, coated with a membrane configured for form the cavity of the chamber. Alternatively, the chamber body 4 can be formed of a folded polymeric material with or without supporting ribs.

The concertina folded body 4 can employ a similar actuation mechanism as the actuation mechanism described with reference to Figs. 2A and 2B: a catheter 14 affixed to the proximal end of the chamber body 4 at the proximal fixation point P1, and pull- and/or push-wire 16 affixed to the distal end of the chamber body 4 at the distal fixation point P2. However, in contrast to the embodiment described with reference to Figs. 2A and 2B, the concertina body 4 expands to increase its interior volume as the distance D between the proximal and distal fixation points P1, P2 increases (see Fig. 3A), whereas a decrease in the distance D between fixation points P1 and P2 leads to a decrease in the internal volume of the chamber as the chamber body 4 collapses (see Fig. 3B).

The embodiment shown in Figs. 3A and 3B comprises a neck 18, similar to the embodiments described with reference to Figs. 2A and 2B. However the skilled person will appreciate that the neck 18 can be omitted, and replaced with an opening 8 in the membrane 12 as shown in Figs. 4A and 4B. A one-way outlet valve can be provided at the outlet opening 8 and one or more one-way inlet valves can be provided in the membrane 12 of the chamber body 4.

The concertina folded body 4 can be biased into the expanded configuration shown in Fig. 3A, and compressed with a similar pull-wire and catheter mechanism as described with reference to Figs. 2A and 2B. Alternatively, the concertina body can be biased into the collapsed configuration (shown in Fig. 3B) and actuated with a push-wire. The wire 16 can also be configured as a push and pull wire that allows the body to be actively collapsed and expanded between the positions shown in Figs. 3A and 3B.

To allow minimally invasive placement within the heart (or an associated lumen, such as the aorta) before being expanded to the position shown in Figs. 3A and 3B, the body 4 can be supported by a support frame or structure. The support frame or structure can comprise a series of resiliently deformable support rings 20, 20', secured to the membrane 12. The support rings 20, 20' can vary in size, alternating between a larger support ring 20 and a smaller support ring 20', to form the concertina shaped body 4. The support rings 20, 20' can be made of a resiliently deformable material or a shape memory material, such that they can be deformed to allow insertion of the device 2 in a minimally invasive manner (e.g. percutaneously), before expanding to a deployed position (shown in Figs. 3A and 3B) in the heart. Although resiliently deformable support rings 20, 20' (e.g. formed of a shape memory material such as nitinol) can allow for convenient insertion, advancement and deployment of the device 2, it will be appreciated that the material that forms the concertina body can be chosen and formed to provide accordion folds in the chamber body 4 without the need for support rings 20, 20'. Moreover, the support structure in concertina-folded embodiments can also comprise a continuous spiral of resiliently deformable wire, which can be compressed and expanded as shown in Figs. 3A and 3B.

The support structure can also comprise an expandable lattice, for example of the type used in (self-expandable) Trans-catheter Aortic Valve Implants (TAVI), wherein the lattice forms the support structure that supports the membrane 12 of the present invention.

It will be appreciated that the embodiments described with reference to Figs. 2A-2B and 3A-3B are actuated by varying the length D of the chamber body 4. In the embodiments described above, a pull- and/or push-wire 16 and a catheter 14 can be moved longitudinally relative to each other in an axial direction to vary the distance D between the first and second fixation points.

However, alternative systems for varying the distance D between the first and second fixation points also fall within the scope of the present invention. For example, the first and second fixation points can be connected to each other by a component having a variable length D. Therefore, rather than relying up relative movement between the catheter and the wire to vary the distance D between D1 and D2, the connecting component can vary its length between D1 and D2, thus resulting the collapse and expansion shown in Figs. 2A-2B and 3A-3B. The connecting component can comprise a spring, a telescopic component, etc. and may be controlled pneumatically, electrically, or by other means.

Referring now to Fig. 4A and 4B, the expansion and contraction of the chamber can also be controlled by relative rotation between the proximal and distal fixation points P1, P2 as an alternative or in addition to relative axial movement (i.e. translational movement along the longitudinal axis, and preferably linear translational movement). As shown in Fig. 4A and 4B, the device 2 can comprise a chamber body 4 similar to the chamber body shown in Figs. 2A and 2B. However, in the embodiment shown in Figs. 4A and 4B, the support ribs 10 are configured as collapsible scaffolding which is configured to expand and collapse the chamber body 4 as the proximal fixation point P1 is twisted with respect to the distal fixation point P2. As shown in Figs. 4A and 4B, when the proximal fixation point P1 is twisted with respect to the second fixation point P2, the chamber body 4 is twisted or subjected to torsion about its longitudinal axis, thereby restricting its maximum cross-sectional diameter, and reducing its internal volume accordingly (see Fig. 4B). Twisting the scaffolding in the opposite direction unwinds the support ribs 10, allowing the chamber body 4 to expand to its maximum cross-sectional diameter (shown in Fig. 4A), and increasing its internal volume accordingly. Alternating the direction of the rotation between the distal and proximal fixation points P1 and P2 thus allows the chamber body 4 to be alternately expanded and collapsed to pump blood through the outlet opening 8 to provide circulatory assistance. The relative rotation of the proximal and distal fixation points P1 and P2 can be actively controlled in both directions (clockwise and anti-clockwise) or the support ribs 10 can be biased towards the position shown in Fig. 4A or the position shown in Fig. 4B, and the twisting of the proximal distal fixation point P1 in a first direction relative to the distal fixation point P2 can act against the bias of the ribs 10 to deform the chamber body 4.

The actuation mechanism configured to twist the proximal and distal fixation points P1 and P2 relative to each other can be similar to the actuation mechanism of the push/pull embodiments described above, except instead of relative axial movement (i.e. translational movement back and forth along the longitudinal axis A of the of the device), the catheter 14 and the wire 16 are configured for relative rotational movement (i.e. wherein one of the catheter 14 and the wire is configured to rotate about the longitudinal axis A, whilst the other remains fixed).

Moreover, in at least some embodiments of the invention the proximal and distal fixation points P1 and P2 can be configured for both axial and rotational movement with respect to each other. For example, the proximal fixation point P1 can be twisted relative to the distal fixation point P2 at the same time as the distance Dx between the fixation points P1 and P2 is decreased to expand the chamber body 4.

In the embodiment shown in Figs. 4A and 4B, the catheter 14 is configured to support the device 2 in a stable position within the heart, whilst the push-rod or wire 16 is configured for rotational movement with respect to the catheter 14 to apply a torsion along the length of the chamber body 4. However, as described above, relative movement between the fixation points P1 and P2 can be achieved with P2 held in a fixed position within the heart chamber, or with neither P1 nor P2 held in a fixed position.

The outlet opening 8 of the embodiment shown in Figs. 4A and 4B differs from the embodiments shown in Figs. 2 and 3 in that it does not comprise a neck. Instead, the opening 8 is formed by leaving an opening at the proximal end of the chamber body that is not covered with the membrane 12. The outlet opening 8 is still relatively narrow compared to the internal diameter of the chamber body at its widest point (along line C-C). However, the skilled person will realise that a neck similar to neck 18 can also be provided in connection with torsion ally collapsible chamber bodies. Moreover, as described above with reference to Figs. 2A and 2B, the outlet opening 8 can also be configured such that it is not relatively narrow compared to the internal diameter of the body. Instead, the outlet opening 8 can be the widest portion of the chamber body 4, or equal in diameter to the widest portion. Moreover, the outlet opening 8 may be configured to expand and collapse with the alternately expanding and collapsing body.

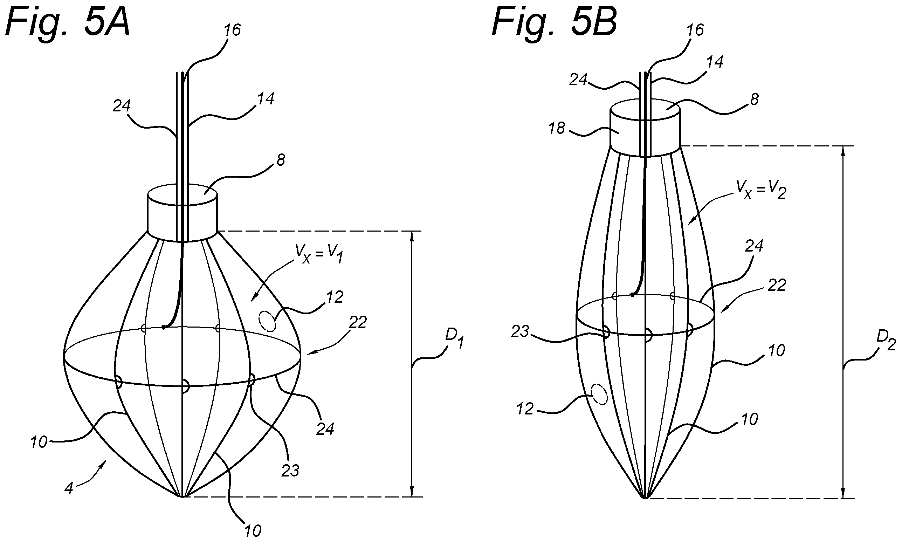

Figs. 5A and 5B show yet another embodiment of the invention comprising a collapsible chamber body 4 for providing circulatory assistance. As shown in Figs. 5A and 5B, the device 2 comprises a chamber body 4 having an outlet opening 8 at a proximal end. The chamber body 4 comprises an internal volume that can be varied by deforming the outer wall of the chamber body 4, thus causing it to alternately collapse and expand to pump blood through the outlet opening 8. The chamber body 4 is biased into an expanded configuration as shown in Fig. 5A. The chamber body 4 can be supported in the expanded configuration by a resiliently deformable scaffold, for example a mesh or a plurality of ribs.

The chamber body 4 can be collapsed (against its bias) into a second collapsed configuration (shown in Fig. 5B) by an actuation mechanism that cinches the chamber body 4 at a cinch point 22 to limit its cross-sectional area, thereby reducing its internal volume. When the tension about the cinch point is released, the chamber body 4 returns to its original expanded configuration under the bias of the resiliently deformable scaffold.

The cinching mechanism can comprise a noose 24 (e.g. a pull-wire noose), which runs around the chamber body 4 at the cinch point 22. To secure the noose 24 in place around the body 4, the noose 24 can run through a channel or a series of eyelets 23, circumferentially positioned about the chamber body 4. To collapse the chamber body 4, the loop or noose 24 is tightened, and the chamber body 4 is restricted at the cinch point 22, thereby reducing the internal volume of the chamber 4, and ejecting blood through the outlet opening 8.

In a related embodiment, instead of using a noose 24 to cinch the chamber body 4 to collapse the chamber body 4 against its bias, a noose 24 can be used as a push wire to expand the chamber a body 4 biased into its collapsed state (Fig. 5B). In such embodiments, the diameter of the noose 24 can be increased by feeding a resilient or stiff wire into the loop formed by the noose 24. Since the noose is coupled to the chamber body 4 (by way of being fed through a channel or a series of eyelets), varying length of wire in the loop of the noose 24 can be used to alternately collapse and expand the chamber body 4.

Yet another embodiment can be based on a similar principle to the cinching noose 24 of Figs. 5A and 5B, except that instead of extending around the chamber body 4 to cinch the cross-sectional diameter of the chamber body 4 in a transverse direction, one or more lengths of wire can be fed though a restricted path (e.g. a channel or through a series of eyelets) secured to the wall of the chamber body 4 in a longitudinal direction. The noose wire 24 can thus be used to expand and contract the chamber body in a longitudinal direction (as opposed to the transverse restriction described with reference to Figs. 5A and 5B.

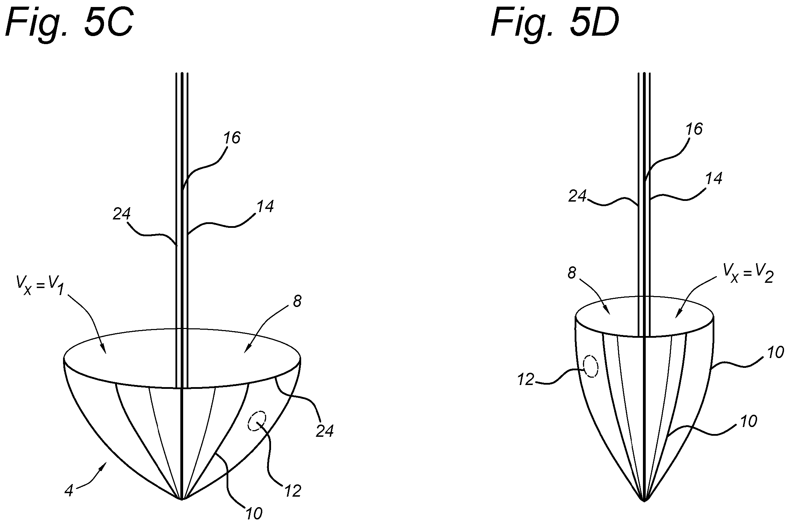

Figs. 5C and 5D illustrate yet another embodiment of the invention, in which a chamber body 4 is collapsed from an expanded configuration to a collapsed configuration. However, in the embodiments shown in Figs. 5C and 5D, the chamber body 4 comprises only the lower part of the chamber body shown in Figs. 5A and 5B. The body 4 comprises a series of struts 10 that are attached to each other at the distal end. The struts are connected to the catheter 14 (or a wire 16) at the distal end of the chamber body and extend radially therefrom. The struts 10 are pivotably connected to the catheter or wire such that an angle between the struts 10 and the catheter 14 can be varied to vary the volume of the chamber body 4. As shown in Figs. 5C and 5D, the chamber body 4 expands and collapses in the manner of an umbrella opening and closing.

For simplicity, the embodiment shown in Figs. 5C and 5D does not show an actuation mechanism. However, the actuation mechanism can comprise a noose, as described with reference to Figs. 5A and 5B, or a sliding strut mechanism, similar to those provided in a conventional umbrella.

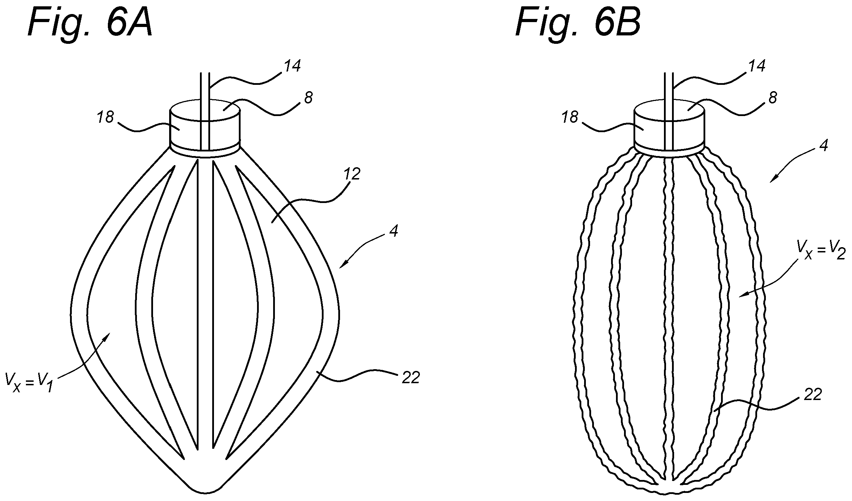

Yet another embodiment of the present invention can comprise an actuation system based upon a system of selectively inflatable support ribs. As shown in Figs. 6A and 6B, the chamber body 4 comprises an outlet opening 8 and is alternately collapsible and expandable between an expanded configuration having a first internal volume (Fig. 6A) and a second collapsed configuration, having a second, smaller internal volume (Fig. 6B). The chamber body is resiliently deformable and is biased into the collapsed state shown in Fig. 6B. The body 4 may be biased into the collapsed position because it is formed of a shape memory material that occupies the configuration shown in Fig. 6B in its undeformed position. The body 4 can comprise a deformable mesh, e.g. made of a shape memory alloy, or a support scaffold comprising a series of ribs or hoops.

The chamber body 4 can further comprise an inflatable scaffold 22. The inflatable scaffold 22 comprises a network of interconnected flexible tubes that, when deflated, do not provide any support to the chamber body 4. However, when inflated, the flexible tubes increase in stiffness to form a scaffold capable of supporting the chamber body 4 in the expanded configuration in shown in Fig. 6A. The inflatable scaffold 22 can be in fluid communication with the catheter 14 to supply an inflating fluid (liquid or air) to the network of tubes.

The inflatable scaffold 22 and the deformable support structure that biases the chamber body 4 into the collapsed configuration can be configured such that the inflatable scaffold 22, when inflated, overcomes the bias of the support structure to expand the chamber body 4, and when the scaffold 22 is deflated, the support structure acts to return the chamber body 4 to the collapsed configuration (See Fig. 6B), thereby reducing the internal volume of the chamber and expelling blood through the outlet opening 6.

Turning now to Figs. 7A-9C, the chamber body 4 itself can take different forms. A longitudinal axis A is defined between the proximal outlet opening 8 and the diametrically opposed distal point of the chamber body 4. The catheter 14 and the wire 16 generally extend along the longitudinal axis A. A transverse axis T extends across the chamber body at its widest point, in a direction that is perpendicular to the longitudinal axis A. Where the chamber body 4 has a generally ellipsoid shape, the major axis of the ellipsoid is the longitudinal axis A, whilst the minor axis is the transverse axis T.

As described above, the chamber body 4 can comprise a deformable support structure covered by a membrane 12, which together form the wall of the chamber body 4. In alterative embodiments, the deformable support structure can be omitted, and the membrane 12 can be self-supporting. Suitable membrane materials include a covered nitinol support structure (e.g. a dip-coated mesh), nylon, polyurethane, Pebax, reinforced compliant polymers and other materials that will be apparent to the person skilled in the art.

The chamber body 4 can also comprise a deformable mesh 26 (e.g. a mesh formed of nickel-titanium shape memory alloy), covered by one or more membrane layers (e.g. membrane 12), or dip-coated to form the chamber wall. It will be appreciated that the support structure 10 illustrated in connected with the above described embodiments can be replaced or augmented by a deformable mesh.

Fig. 7A shows a side view of a chamber body 4 according to at least some embodiments of the invention. As shown in Fig. 7A, the deformable support structure comprises a deformable mesh body 26, formed into a generally ellipsoid shape, the mesh body 26 extending comprising a closed distal end, and an open proximal end. The proximal end of the mesh body 26 is coupled or attached to catheter 14. The coupling between the mesh body 26 and the catheter 14 forms the proximal fixation point P1.

A wire 16 extends through the central lumen of the catheter 14, through the interior of the chamber, and is fixed or coupled to the distal end of the mesh body 26 at a distal fixation point P2. The internal lumen of the catheter 14 is preferably sealed or closed to prevent blood being pumped along the central lumen of the catheter 14.

The mesh body 26 is dip-coated to form a fluid-impermeable layer or membrane over the mesh body, thereby forming the chamber wall. At the proximal end of the mesh body 26, the mesh is uncoated, forming the outlet opening 8 through which blood can be expelled as the chamber is alternately expanded and collapsed. The dip-coated region of the mesh 26 is denoted with reference numeral 26a and the uncoated portion is shown with reference numeral 26b.

In an alternative configuration shown in Figs. 8A-8C, the chamber body 4 can comprise a deformable support structure comprising a scaffold or support structure 10, and a plurality of overlapping panels 12a-f, which together form a membrane that covers the scaffold 10 to form the chamber wall. Fig. 8A shows a side view of the chamber body 4, whilst Fig. 8B and 8C shows cross-sectional views during different phases of the pumping cycle.

As shown in Fig. 8A, the overlapping panels 12a-f are supported on a deformable support structure formed of a plurality of ribs 10. The edges of each of the panels 12a-12f overlaps with the edge of the adjacent panel to form a region of overlap 28 (shown with dotted lines in Fig. 8A).

Turning now to Fig. 8B, it can be seen that the panels 12a-12f can be secured to the ribs 10 on their inner surface, but, along at least a portion of region of overlap, the panels 12a-12f are not secured to each other or the rib on their outer surface. Thus, as shown in Fig. 8B-8C, this leaves an opening 25 between unsecured regions of overlapping panels 12a-12f that is configured to act as an inlet to allow blood to flow into the interior of the chamber.

As shown in Fig. 8B, as the chamber body expands (and the ribs 10 are brought closer together), the pressure within the chamber interior volume Vx is lower than the pressure outside the chamber and blood rushes into the internal volume Vx through the openings 25 between adjacent panels.

However, as shown in Fig. 8C, as the chamber body collapses, the pressure within the chamber body 4 forces the panels 12a-12f into abutment with each other, thereby sealing the openings 25 and forcing blood out of the internal volume Vx and through the outlet opening 8.

The panels 12a-f are configured such that as the chamber body 4, blood from within the heart chamber 4 is drawn into the interior of the chamber 4 through the inlets 25 (this effect is enhanced if the outlet opening 8 is configured as a one-way valve, as described above). However, when the chamber body 4 is collapsed to decrease the internal volume of the chamber, the increase in pressure within the chamber causes the unsecured (but overlapping) regions of the panels 12a-f to seal against each other, thus closing the inlet openings 25, and forcing blood to exit the chamber through the outlet opening 8.

In the embodiment shown in Figs. 8A and 8B, the panels 12a-f form longitudinal regions of overlap such that the inlet openings are formed as longitudinal slits in the chamber body wall (substantially parallel to the longitudinal axis A of the chamber body 4).

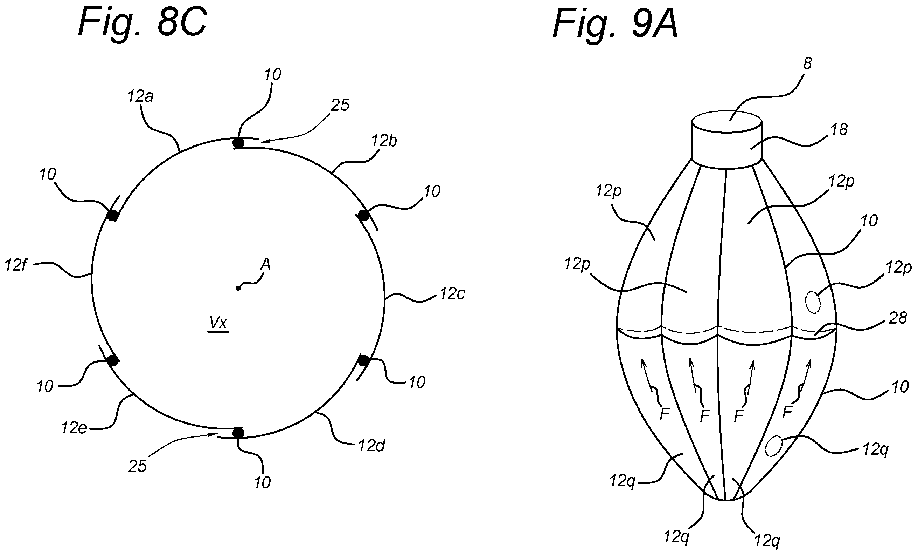

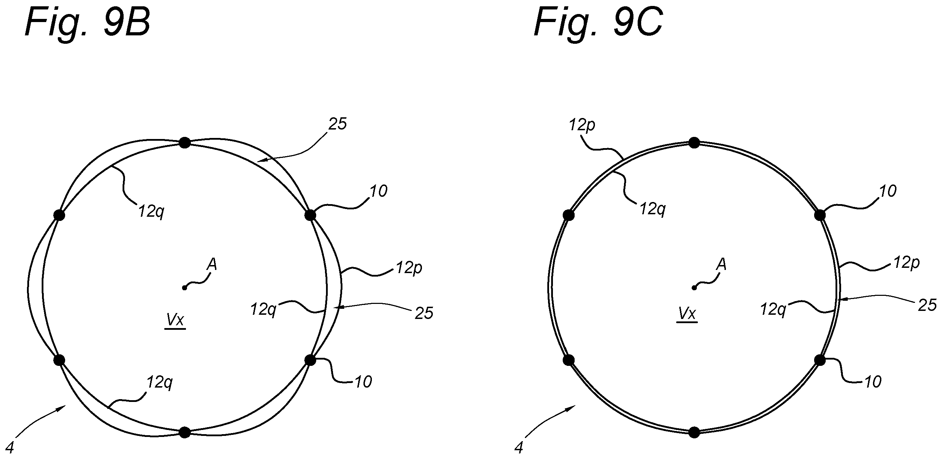

However, as shown in Figs. 9A-9C, in alternative embodiments, the chamber body 4 can also be configured with a one or more proximal panels 12p and one or more distal panels 12q that form regions of overlap 28 that extend in a transverse direction such that the openings or slits formed by unsecured portions of overlapping panels extend in a transverse direction around the circumference of the chamber body 4 (along the lines of latitude of the ellipsoid body). In the embodiment depicted (see Fig. 9A), the proximal panels 12p overlap the distal panels 12q such that the proximal panels 12p extend over an outer surface of the distal panels 12q (when the chamber body 4 is viewed from the outside). This arrangement allow for a fluid flow path through the inlets formed by the overlap in a proximal direction indicated by arrows F in Fig. 9A. This arrangement can ensure that fluid drawn into the internal volume of the chamber body 4 in proximal direction, from the region of the heart chamber that is relatively distant from the proximal opening 8 of the device, minimizing the likelihood that the blood expelled from the chamber through the outlet during systole is drawn back into the chamber during diastole.

Similarly to Figs. 8B and 8C, Figs. 9B and 9C show a cross-sectional view of the chamber body 4 and the openings 25 with the chamber body expanding (see Fig. 9B) and with the chamber body collapsing (see Fig. 9C). As described above with reference to Figs. 8A-8C, the increase in pressure within the internal volume Vx as the chamber body collapses, forces the panels 129 outwardly, and into abutment with the outer panels 12p, thereby sealing the openings 25 and forcing blood from the interior volume Vx out through the opening 8, towards the aorta.

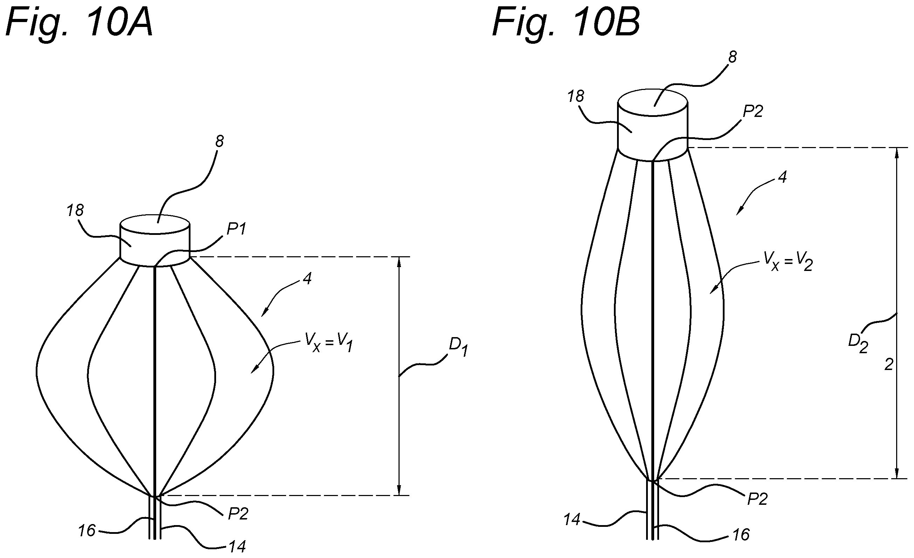

Finally, it will be understood that in each of the embodiments described above, the device 2 is supported and/or actuated by a catheter 14 (or a wire) that extends proximally from the device, in the direction of flow of the fluid ejected through the outlet opening 8. However, it will be appreciated that the support and actuation structures (e.g. the support catheter 14 and the actuation wire 16 can be reversed such that they extend from the opposite end of the device 2). For example, referring now to Figs. 10A and 10B, the device 2 can be configured in a similar manner to Figs. 2A and 2B, however, in the embodiment shown in Figs. 10A and 10B, the catheter 14 is coupled to the distal end of the chamber body 4 at the distal fixation point P2, and the wire 16 extends through the central lumen of the catheter 14 and is coupled to the proximal end of the chamber body 4 at the proximal fixation point P1. For each of the embodiments described, the configuration of the actuation system can be reversed (as shown in Figs. 10A and 10B) to suit the deployment and advancement method desired in a given patient.

The present invention has been described herein with reference to a number of exemplary, nonlimiting embodiments. The skilled person will realize that features from the embodiments described herein can be combined, and modifications can be made without departing from the scope of the invention.

### Further Embodiments

A heart support device 2 for circulatory assistance is provided, the device comprising:a chamber body 4 comprising an outer wall 12 and defining an internal volume Vx configured to receive a volume of fluid, wherein the chamber body 4 extends from a proximal end 4a to a distal end 4b, wherein the chamber body 4 comprises an outlet opening 8 at its proximal end 4a, the outlet opening 8 being in fluid communication with an exterior volume in which the chamber body 4 is disposed, wherein the outer wall of the chamber body 4 is configured to alternately collapse and expand between a first configuration in which the internal volume Vx=Vi and a second configuration in which the internal volume V_{X}=V2, wherein Vi is larger than V2, and thereby pump the fluid through the outlet opening 8, and wherein the outlet opening 8 comprising a cross-sectional internal diameter, and wherein the cross-sectional internal diameter of the outlet opening 8 is less than a maximum cross- sectional internal diameter of the chamber body 4.

The chamber body 4may further comprise a deformable support structure 10 configured to support the chamber body 4 in an open configuration.

The chamber body 4 may be biased into the second, collapsed configuration, and wherein the device 2 further comprises an actuation system 14, 16, 22, 24 configured to expand the chamber body 4 against its bias.

The chamber body 4 may be biased into the first, expanded configuration, and wherein the device 2 further comprises an actuation system 14, 16, 233, 24 configured to collapse the chamber body 4 against its bias.

The actuation system 14, 16 may further comprise a first actuation catheter or wire 14 coupled to a proximal end of the chamber body 4 at a proximal fixation point P1 and a second actuation catheter or wire 16 coupled to a distal end of the chamber body 4 at a distal fixation point P2, and wherein first the actuation catheter or wire 14 is configured for relative movement with respect to the second actuation wire or catheter 16 to move the proximal fixation point P1 back and forth relative to the distal fixation point P2 to alternately collapse and expand the chamber body 4.

The first actuation wire or catheter 14 and the second actuation wire or catheter 16 may be configured for rotational movement with respect to each other to twist the chamber body 4 along its axis.

The first actuation wire or catheter 14 and the second actuation wire or catheter 16 may be configured for translational movement towards and away from each other to vary the length of the chamber body 4

The chamber body 4 may comprise a concertina folded outer wall.

The actuation system may comprise one or more inflatable ribs 22, said ribs, upon inflation, being configured to act against the bias of the chamber body 4 to expand or collapse the chamber body 4.

The device 2 may comprise a catheter 14 affixed to the chamber body 4, the catheter 14 comprising an internal lumen in fluid communication with the inflatable ribs 22.

The device may comprise an actuation system configured to collapse and expand the body, and wherein the actuation system comprises a noose 24 extending around a circumference of the chamber body 4, and wherein the noose 24 comprises a variable loop, and wherein the loop is coupled to the support structure 10 such that tightening the noose 24 cinches the support structure 10 at a cinch point 22 to restrict the volume of the chamber body 4.

The chamber body 4 may be biased into the expanded configuration, and wherein the actuation system comprises a noose 24 configured to collapse the chamber body 4.

The heart support device may further comprise at least one inlet opening 24 in the outer wall of the chamber body 4 to provide fluid communication between the interior volume Vx of the chamber body 4 and a volume exterior to the device.

The inlet opening 24 is closed by a one way valve.

The chamber body 4 may comprise a plurality of deformable ribs 10 forming a support scaffold; and one or more flexible panels or membranes 12 supported by the deformable ribs 10 to form the chamber body 4.

The chamber body 4 may comprise a plurality of overlapping panels 12a-d, 12p-q, and wherein at least one inlet opening 25 is provided between the between overlapping panels 12a-d, 12p-q.

The outlet opening 8 may comprise a one-way valve.

The one-way valve may comprise a collapsible neck.

The support structure 10 may comprise a frame comprising a shape memory material.

The support structure 10 may comprise a plurality of helically collapsible ribs.

## Claims

1. A heart support device for circulatory assistance, the device comprising:
a chamber body comprising an outer wall and defining an internal volume (Vₓ) configured to receive a volume of fluid, wherein the chamber body extends from a proximal end to a distal end and is configured to be at least partially placed in a heart chamber; and
a catheter extending from the proximal end of the chamber body,
wherein the chamber body comprises an elongated portion having an outlet opening at the proximal end of the chamber body, the elongated portion configured to extend through a heart valve and into a vessel adjacent the heart chamber when the chamber body is placed in the heart chamber, the outlet opening being in fluid communication with an exterior volume in which the chamber body is disposed,
wherein the chamber body further comprises an inlet opening in the outer wall of the chamber body to provide fluid communication between the internal volume of the chamber body and the exterior volume, and
wherein the outer wall of the chamber body is configured to alternately collapse and expand between a first configuration in which the internal volume Vₓ=V₁ and a second configuration in which the internal volume Vₓ=V₂, wherein V₁ is larger than V₂, and thereby pump the fluid through the outlet opening.

2. The heart support device according to claim 1, wherein the chamber body further comprises a deformable support structure configured to support the chamber body in an open configuration.

3. The heart support device according to claim 2, wherein the support structure comprises a frame comprising a shape memory material.

4. The heart support device according to any one of claims 1-3, wherein the chamber body is biased into the second, collapsed configuration, and wherein the device further comprises an actuation system configured to expand the chamber body against its bias.

5. The heart support device according to claim 4, wherein the actuation system comprises one or more inflatable ribs, said ribs, upon inflation, being configured to act against the bias of the chamber body to expand or collapse the chamber body.

6. The heart support device according to claim 5, wherein the device comprises a catheter affixed to the chamber body, the catheter comprising an internal lumen in fluid communication with the inflatable ribs.

7. The heart support device according to any one of claims 1-3, wherein the chamber body is biased into the first, expanded configuration, and wherein the device further comprises an actuation system configured to collapse the chamber body against its bias.

8. The heart support device according to any one of claims 1-7, wherein the device comprises an actuation system comprising a first actuation catheter or wire coupled to a proximal end of the chamber body at a proximal fixation point and a second actuation catheter or wire coupled to a distal end of the chamber body at a distal fixation point, and wherein first the actuation catheter or wire is configured for relative movement with respect to the second actuation wire or catheter to move the proximal fixation point back and forth relative to the distal fixation point to alternately collapse and expand the chamber body.

9. The heart support device according to claim 8, wherein the first actuation wire or catheter and the second actuation wire or catheter are configured for rotational movement with respect to each other to twist the chamber body along its axis.

10. The heart support device according to claim 8, wherein the first actuation wire or catheter and the second actuation wire or catheter are configured for translational movement towards and away from each other to vary the length of the chamber body.

11. The heart support device according to claim 10, wherein the chamber body comprises a concertina folded outer wall.

12. The heart support device according to any one of claims 1-3, wherein the device comprises an actuation system configured to collapse and expand the body, and wherein the actuation system comprises a noose extending around a circumference of the chamber body.

13. The heart support device according to claim 1, wherein the inlet opening is closed by a one way valve.

14. The heart support device according to claim 1, wherein the outer wall of the chamber body comprises a mesh and one or more membrane layers.

15. The heart support device according to claim 1, wherein the heart chamber is a left ventricle.
